# EUROPÄISCHE PATENTSCHRIFT

(11) **EP 3 074 747 B1**
(45) Veröffentlichungstag und Bekanntmachung des Hinweises auf die Patenterteilung: **05.10.2022**
(21) Anmeldenummer: 14755346.5
(22) Anmeldetag: 15.08.2014
(51) Int. Cl.: G01N 3/40, G01N 33/15

(54) **VORRICHTUNG UND VERFAHREN ZUR PRÜFUNG VON TABLETTEN**
DEVICE AND METHOD FOR TESTING TABLETS
DISPOSITIF ET PROCÉDÉ POUR LE CONTRÔLE DE COMPRIMÉS

(30) Priorität: 27.11.2013 DE 102013113126
(43) Veröffentlichungstag der Anmeldung: 05.10.2016
(73) Patentinhaber: Erweka GmbH, 63225 Langen (DE)
(72) Erfinder: MÜLLER, Werner G., 63150 Heusenstamm (DE); BOZKURT, Levent, 63150 Heusenstamm (DE)
(74) Vertreter: Meyer-Dulheuer MD Legal Patentanwälte PartG mbB
(86) Internationale Anmeldenummer: PCT/EP2014/067506
(87) Internationale Veröffentlichungsnummer: WO 2015/078600

(56) Entgegenhaltungen:
- EP-A1- 2 587 261
- WO-A1-89/07083
- WO-A1-2013/083308
- WO-A2-2013/061223
- US-A1- 2005 103 132

## Beschreibung

Gegenstand der vorliegenden Erfindung sind eine Vorrichtung und ein Verfahren zur Prüfung von Tabletten. Im Rahmen der Qualitätskontrolle von Tabletten ist es notwendig, diese auf ihre Eigenschaften wie Länge, Breite, Bruchfestigkeit und Gewicht zu überprüfen. Das Deutsche Arzneibuch enthält die hierfür relevanten gesetzlichen Bestimmungen. Dieser Vorgang sollte nach Möglichkeit automatisch vonstattengehen, sodass eine Vielzahl von Tabletten innerhalb kurzer Zeit überprüft werden kann. Zudem muss gewährleistet werden, dass Tabletten unterschiedlicher Formen und Größen richtig positioniert werden können, um eine fehlerfreie Messung zu ermöglichen. Der Stand der Technik kennt mehrere Vorrichtungen und Verfahren, die hierfür geeignet sind.

Die Deutsche Patentschrift DE 197 33 436 C2 beschreibt ein Tablettentestgerät zum Test von Oblongtabletten. Hierbei werden die zu prüfenden Tabletten durch eine Zufuhreinrichtung zunächst einer Waage zugeführt und von dieser anschließend auf eine Transporteinrichtung verbracht. Auf der Transporteinrichtung werden die Tabletten hierbei in einer definierten Position abgelegt, sodass weitere Tests stattfinden können. Die korrekte Ausrichtung der Tablette bei Ablage auf der Transporteinrichtung wird hierbei dadurch bewirkt, dass die Waagschale einen Boden in Form einer Rinne aufweist. Dieser sorgt dafür, dass die Oblong-Tabletten eine entsprechende Ausrichtung erfahren. Darüber hinaus kann sich auf der Transporteinrichtung ein Stolperstein, beispielsweise ein Keil, befinden, der für den Fall vorgesehen ist, dass die Tablette mit ihrer Kopfseite auf die Transportvorrichtung fällt. In diesem Fall sorgt der Stolperstein dafür, dass die Tablette auf ihre lange Seite kippt. Gemäß dieser Erfindung wird eine korrekte Ausrichtung einer Tablette bewirkt, jedoch ist die Tablette bei ihrer Übergabe durch die Rinne nicht unerheblichen Kräften ausgesetzt, die zu ihrer Beschädigung führen könnten.

Die Deutsche Patentschrift DE 10 2006 004 215 B4 offenbart einen Bruchfestigkeitstester für Tabletten unterschiedlicher Form und Größe. Um die Ausrichtung der Tabletten zur bewerkstelligen, werden hier zwei gegenläufig angetriebene, nebeneinander angeordnete Zentrierrollen eingesetzt. Wird eine Oblong-Tablette auf den Rollen platziert, so sorgt deren Rotation dafür, dass sich die Oblong-Tablette in Längsrichtung zwischen den Rollen ausrichtet. Anschließend kann eine Überprüfung der Festigkeit durch eine Bruchbacke erfolgen. Um die Ausrichtbarkeit von Tabletten unterschiedlicher Form und Größe zu gewährleisten, ist die Vorrichtung mit einem schwenkbaren Positionierer ausgestattet, der die Rollen unterschiedlich ausrichten kann. Die Ausrichtung geht schnell vonstatten, die Apparatur könnte aufgrund der Verwendung von Rollen nach einem erfolgten Bruchtest allerdings schwer zu reinigen sein.

Die Gebrauchsmusterschrift DE 298 24 199 U1 offenbart ein System zur Durchführung von Härtetests an Prüfkörpern, die einen alternativen Ansatz verfolgt. Hierbei wird die Tablette auf einem Prüftisch platziert, aber daraufhin wird keine Ausrichtung der Tablette durchgeführt. Stattdessen wird durch eine Vorrichtung zur Lageerkennung die Ausrichtung des Prüfkörpers festgestellt. Daraufhin werden ein Druckkolben sowie ein Gegenlager, die zum Härtetest der Tablette dienen, entsprechend ausgerichtet, damit die Überprüfung der Tablette erfolgen kann. Diese Erfindung setzt jedoch einen komplizierten und kostspieligen Aufbau voraus, der Mittel zur Bilderkennung umfassen muss.

Die PCT-Veröffentlichungsschrift WO 2013/061223 A2 offenbart ein Verfahren und eine Vorrichtung zur Überprüfung von Tabletten, das die Tabletten unter Zuhilfenahme einer schwenkbaren Wippe ausrichtet. Die Tablette wird hierbei auf die zunächst waagerechte Wippe verbracht, die nun eine Schwenkbewegung durchführt. Durch die Wirkung der Schwerkraft und der Schwenkbewegung wird bewirkt, dass die Tablette sich an einer Positionierungsfläche ausrichtet. Die Positionierungsfläche hat hierbei eine konkave Form, die entlang der durch die Bewegung der Wippe definierten Form verläuft. Um die korrekte Ausrichtung der Tablette zu unterstützen, kann die schwenkbare Wippe zusätzlich in einem Winkel zur Positionierungsfläche geneigt sein, oder aber eine zusätzliche Einrichtung auf der Seite gegenüberliegend der Positionierungsfläche besitzen, welche eine Vorausrichtung der Tablette vornimmt. Nach erfolgter Positionierung und Rückkehr der Wippe in eine waagerechte Position erfolgt die Überprüfung der Tablette. Da die Positionierung hier unter Einwirkung der Schwerkraft geschieht und die Wippbewegung mit einer ausreichenden Geschwindigkeit durchgeführt werden muss, kann auch hier nicht ausgeschlossen werden, dass die auf die Tablette wirkenden Kräfte bei der Ausrichtung zu hoch sein können. Dokument WO 89/07083 A1 offenbart den kennzeichnenden Teil der Ansprüche 1 und 8.

Aufgabe der vorliegenden Erfindung ist es daher, eine Vorrichtung und ein Verfahren zur Überprüfung von Tabletten bereitzustellen, das die Tablette vor ihrer Überprüfung in einer schonenden, schnellen und einfach zu implementierenden Weise ausrichtet, wobei auch eine problemlose Reinigbarkeit gewährleistet sein soll.

Gelöst wird die Aufgabe durch eine Vorrichtung und ein Verfahren gemäß der Ansprüche 1 und 8. Die Vorrichtung umfasst eine Drehscheibe (1), die dazu eingerichtet ist, eine Tablette (7) zur Prüfung zu positionieren. Mindestens eine Positionierungsfläche (2a, 3a) befindet sich oberhalb der Drehscheibe (1), entlang welcher die Tablette (7) durch Rotation der Drehscheibe (1) in einer Rotationsrichtung positionierbar ist.

Eine Positionierungsfläche (2a) ist als Widerlager (2) ausgeführt. Durch Rotation der Drehscheibe (1) wird die Tablette (7) an das Widerlager (2) herangeführt und entlang dem Widerlager (2) positioniert.

Bei der Tablette handelt es sich vorzugsweise um eine Oblong-Tablette, die sich aufgrund ihrer Form ihrer Länge nach an der Positionierungsfläche (2a, 3a) ausrichtet. Es ist jedoch auch möglich, Tabletten anderer Form zu positionieren, beispielsweise runde Tabletten. Hierbei führt der Vorgang lediglich dazu, dass die Tablette an die Positionierungsfläche (2a, 3a) herangeführt wird, sodass eine Prüfung durchgeführt werden kann. Bei den nachfolgenden Ausführungen ist zu beachten, dass der Prüfvorgang ohne menschliches Zutun abläuft. So wird beispielsweise die Drehscheibe (1) von einem Motor angetrieben. Vorzugsweise verfügt die Vorrichtung über eine Rechenanlage, die nicht nur die Bewegungsabläufe steuert, sondern auch die gemessenen Werte speichert.

Gemäß der Erfindung ist eine weitere Positionierungsfläche (3a) vorhanden, die als Anschlag (3) ausgeführt wird. Der Anschlag (3) kann in einem beliebigen Winkel zum Widerlager (2) angeordnet sein, vorzugsweise ist er jedoch in einem rechten Winkel zum Widerlager (2) angeordnet. Eine Ausrichtung der Tablette (7) am Anschlag (3) erfolgt, wenn sich die Drehscheibe (1) in einer Rotationsrichtung dreht, die entgegengesetzt zu der Rotationsrichtung ist, die dazu führt, dass die Tablette (7) am Widerlager ausgerichtet wird.

Vorzugsweise führt die Drehscheibe (1) ihre Rotationsbewegung fort, auch nachdem bereits eine Ausrichtung der Tablette (7) an der jeweiligen Positionierungsfläche (2a, 3a) erfolgt ist. Es ist hierbei besonders vorteilhaft, wenn die Rotationsbewegung auch während der Prüfung der Tablette (7) fortgeführt wird.

Ist es nicht gewünscht, dass die Rotation der Drehscheibe (1) während der Ausrichtung der Tablette (7) am Widerlager oder Anschlag fortgeführt wird, so ist es gemäß einer weiteren Ausführungsform möglich, dass die Rotation der Drehscheibe (1) ausgesetzt wird, sobald eine erfolgreiche Ausrichtung erfolgt ist. Die Aussetzung der Rotation kann nach dem Ablauf einer gewissen Zeitspanne erfolgen, die für eine erfolgreiche Ausrichtung der Tablette (7) unter gewöhnlichen Umständen hinreichend ist. Alternativ kann durch ein Sensorsystem überprüft werden, ob die Ausrichtung der Tablette (7) erfolgreich gewesen ist, und zu diesem Zeitpunkt die Rotation der Drehscheibe (1) ausgesetzt werden. Ein hierfür geeignetes Sensorsystem kann beispielsweise durch ein Kamerasystem implementiert werden.

Widerlager (2) und Anschlag (3) sind als ebene Flächen ausgeführt, die senkrecht über der Drehscheibe (1) positioniert sind, wobei ein möglichst kleiner Abstand zwischen Drehscheibe (1) und der jeweiligen Positionierungsfläche (2a, 3a) besteht. Der Abstand zwischen Drehscheibe (1) und der jeweiligen Positionierungsfläche (2a, 3a) darf nicht so groß sein, dass eine zu testende Tablette (7) unter die Positionierungsfläche (2a, 3a) geraten kann und somit nicht mehr an der Positionierungsfläche (2a, 3a) zum Liegen kommen und deshalb nicht an dieser durch die Drehung der Drehscheibe (1) ausgerichtet werden kann.

Widerlager (2) und Anschlag (3) sollten zueinander in einem rechten Winkel ausgerichtet sein, können aber auch in einem anderen Winkel zueinander ausgerichtet sein. Die Positionierungsflächen (2a, 3a) werden relativ zur Drehscheibe (1) so angebracht, dass die gedachte Linie, die durch die jeweilige Positionierungsfläche (2a, 3a) definiert wird, jeweils möglichst nahe zum Mittelpunkt der Drehscheibe (1) oder bestenfalls durch deren Mittelpunkt verläuft.

Um eine bessere Positionierung der Tablette entlang den Positionierungsflächen (2a, 3a) zu ermöglichen, können die Positionierungsflächen (2a, 3a) eine besondere Form aufweisen. Erfindungsgemäß wird eine ebene Form als besonders vorteilhaft angesehen. Es ist jedoch auch möglich, die Positionierungsflächen (2a, 3a) in einer konkaven Form auszugestalten. Es ist außerdem möglich, die Positionierungsflächen (2a, 3a) mit einer aufgerauten Oberfläche oder einem speziellen Muster zu versehen, welche eine bessere Ausrichtung der Tabletten erlauben.

Ebenso kann die Oberfläche der Drehscheibe (1) eine besondere Ausprägung besitzen, um einen besseren Transport der Tablette (7) zu ermöglichen. So ist hier eine leicht raue Oberfläche als vorteilhaft zu erachten.

In einer weiteren Ausführungsform ist in die Drehscheibe (1) oder unter der Drehscheibe eine Waage integriert. Diese ist dazu in der Lage, das Gewicht der auf die Drehscheibe (1) aufgebrachten Tablette (7) zu bestimmen.

Weiterhin ist es erfindungsgemäß, dass die Vorrichtung über eine Bruchbacke (4) verfügt, die in Richtung des Widerlagers (2) bewegbar ist. Mithilfe der Bruchbacke (4) kann der Durchmesser der Tablette (7) gemessen werden, welche entlang der mindestens einen Positionierungsfläche (2a, 3a) ausgerichtet ist, wobei der Durchmesser in Richtung der Bewegungsrichtung der Bruchbacke (4) gemessen wird. Handelt es sich bei der Tablette (7) um eine Oblong-Tablette, so wird zunächst die Breite der Tablette (7) gemessen, und anschließend in einer zweiten Position die Länge.

Bevorzugt ist die Bruchbacke (4) weiterhin dazu geeignet, eine Überprüfung der Bruchhärte einer Tablette (7) durchzuführen, welche entlang der mindestens einen Positionierungsfläche (2a, 3a) ausgerichtet ist.

In einer bevorzugten Ausgestaltung der Vorrichtung ist ein Rechen (6a) vorgesehen, der das Tablettenmaterial nach erfolgter Prüfung von der Drehscheibe (1) schiebt.

Ist die weitere Positionierungsfläche (3a) als Anschlag (3) ausgeführt, ist es vorteilhaft, dass der Anschlag (3) an einem Drehgelenk (8) mit Feder befestigt ist, der dazu vorgesehen ist, einer Bewegung durch den Rechen (6a) nachzugeben und eigenständig in seine ursprüngliche Position zurückzukehren.

Es ist zudem bevorzugt, dass es sich bei dem Rechen (6a) um einen Transportrechen (6) handelt, mit dem die Tablette (7) vor der Überprüfung auf die Drehscheibe (1) schiebbar ist. Der Transportrechen (6) kann somit sowohl die Tablette (7) auf die Drehscheibe (1) transportieren, als auch nach erfolgter Prüfung die Tablette oder Tablettenreste von der Drehscheibe (1) schieben.

Weiterhin ist ein Verfahren zur Prüfung von Tabletten Gegenstand der vorliegenden Erfindung.

Das erfindungsgemäße Verfahren zur Prüfung von Tabletten beinhaltet mindestens die folgenden Schritte. Eine Tablette (7), vorzugsweise eine Oblong-Tablette, wird auf die Drehscheibe (1) verbracht. Die Tablette (7) kommt durch die Drehung der Drehscheibe (1) in Kontakt mit einer Positionierungsfläche (2a, 3a) und wird an dieser ausgerichtet. Die Ausrichtung der Tablette (7) erfolgt dadurch, dass diese durch die Drehung der Drehscheibe (1) in Kontakt mit einer Positionierungsfläche (2a, 3a) kommt und an dieser Positionierungsfläche (2a, 3a) aufgrund der Drehung der Drehscheibe (1) entlang ihrer Längsachse ausgerichtet wird. Dabei kann die Drehung der Drehscheibe (1) bereits erfolgen, bevor die Tablette (7) auf die Drehscheibe (1) gebracht wird, oder erst beginnen, nachdem sich die Tablette (7) auf der Drehscheibe (1) befindet. Anschließend findet mindestens eine Prüfung der Tablette (7) statt.

Gemäß der Erfindung wird zur Prüfung der Tablette (7) in einer ersten Position diese dadurch positioniert, dass die Drehscheibe (1) in einer ersten Richtung rotiert, sodass die Tablette (7) entlang einer Positionierungsfläche (2a), die ein Widerlager (2) ist, ausgerichtet wird. Um eine weitere Prüfung durchzuführen, wird die Tablette (7) zur Prüfung in einer weiteren Position dadurch positioniert, dass die Drehscheibe (1) in einer Richtung entgegengesetzt der Drehrichtung der vorherigen Drehung rotiert, sodass die Tablette (7) auf eine Positionierungsfläche (3a), die ein Anschlag (3) ist, trifft und entlang dieser ausgerichtet wird. Handelt es sich bei der Tablette (7) um eine Oblong-Tablette, so wird zunächst in einer ersten Position die Breite der Tablette (7) gemessen und anschließend in einer zweiten Position die Länge. Nach erfolgter Längenmessung erfolgt der Bruchtest in der derselben Position.

Bevorzugt wird das erfindungsgemäße Verfahren wie folgt durchgeführt:
1. Eine Tablette (7), vorzugsweise eine Oblong-Tablette, wird auf die Drehscheibe (1) verbracht.
2. Die Tablette (7) wird durch die Drehung der Drehscheibe (1) an einer ersten Positionierungsfläche (2a) ausgerichtet.
3. Eine Prüfung der Breite der Tablette (7) wird durchgeführt.
4. Die Tablette wird durch die Drehung der Drehscheibe (1), die in eine Drehrichtung entgegengesetzt zur Drehrichtung bei der ersten Positionierung erfolgt, an einer weiteren Positionierungsfläche (3a) ausgerichtet.
5. Eine Prüfung der Länge der Tablette (7) wird durchgeführt.
6. Ein Bruchtest der Tablette (7) wird durchgeführt.
7. Die Überreste der Tablette (7) werden von der Drehscheibe (1) entfernt.

Abhängig von den angestrebten Prüfzielen ist aber auch alternativ ein anderer Ablauf des Verfahrens möglich: so beispielsweise eine Variante, bei der nur eine Messung der Durchmesser, aber kein Bruchtest durchgeführt wird. Der Begriff Durchmessermessung umfasst im Sinne der vorliegenden Erfindung eine Breiten- und/oder Längenmessung der Tablette.

Im Folgenden wird beschrieben, auf weiche Weise Durchmesser und Bruchhärte der Tablette (7) geprüft werden können, wie die Tablette (7) auf die Drehscheibe (1) verbracht werden kann, und welche Möglichkeiten bestehen, um die Tablette (7) oder deren Überreste nach der Prüfung von der Drehscheibe (1) zu entfernen.

Die Prüfung der Tablette (7) wird durch eine Bruchbacke (4) durchgeführt. Die Bruchbacke (4) vollzieht eine Bewegung in Richtung des Widerlagers (2). Sie wird durch einen Motor angetrieben. Die Bruchbacke (4) ist dazu ausgelegt, den Durchmesser der Tablette (7) sowie deren Bruchhärte zu überprüfen. Die Überprüfung der Dimensionen erfolgt hierbei dadurch, dass die Bruchbacke (4) so lange in Richtung des Widerlagers (2) bewegt wird, bis eine definierte Gegenkraft auftritt. Diese Gegenkraft zeigt auf, dass die Bruchbacke (4) auf die Tablette (7) getroffen ist. Der zurückgelegte Weg der Bruchbacke (4) wird gespeichert, wobei optional hieraus direkt der Durchmesser der Tablette (7) errechnet wird. Der Durchmesser der Tablette (7) berechnet sich aus dem maximal zurücklegbaren Weg zwischen der Ausgangsposition der Bruchbacke (4) und dem Widerlager (2) abzüglich des tatsächlich zurückgelegten Wegs der Bruchbacke.

Die Messung des Durchmessers kann entweder erfolgen, nachdem die Tablette (7) entlang dem Widerlager (2) positioniert worden ist, oder aber nachdem die Tablette (7) entlang dem Anschlag (3) ausgerichtet worden ist. Ist eine Positionierung entlang dem Widerlager (2) durchgeführt worden, so erfolgt durch die Bruchbacke (4) eine Messung des Breitendurchmessers. Ist eine Positionierung entlang dem Anschlag (3) durchgeführt worden, so erfolgt durch die Bruchbacke (4) eine Messung des Längsdurchmessers der Tablette (7).

Gemäß der Erfindung ist die Bruchbacke (4) dazu eingerichtet, eine Messung der Bruchhärte der Tablette (7) durchzuführen. Zu diesem Zweck wird die Bruchbacke (4) so lange in Richtung des Widerlagers bewegt, bis auf die Bruchbacke (4) eine definierte Gegenkraft auftritt. Dies zeigt an, dass die Bruchbacke (4) die Tablette (7) erreicht hat. Daraufhin wird die durch die Bruchbacke (4) angelegte Kraft so lange gesteigert, bis die Tablette (7) zerbricht. Die durch die Bruchbacke (4) angelegte Kraft, die notwendig war, um einen Bruch der Tablette (7) herbeizuführen, wird hierbei durch die integrierte Rechenanlage der Vorrichtung gespeichert. Die Messung der Bruchhärte kann nur erfolgen, wenn die Tablette (7) zuvor an einer Positionierungsfläche (2a, 3a) ausgerichtet worden ist. Ist die Ausrichtung am Widerlager (2) durchgeführt worden, so erfolgt eine Prüfung der Bruchhärte bezogen auf die lange Seite der Tablette (7). Ist die Ausrichtung am Anschlag (3) durchgeführt worden, so erfolgt eine Prüfung der Bruchhärte bezogen auf die kurze Seite der Tablette (7).

In einer weiteren Ausführungsform wird die Tablette (7) beziehungsweise werden deren Überreste nach erfolgter Prüfung durch einen Rechen (6a) von der Drehscheibe (1) geschoben. Der Rechen (6a) führt in einer vorteilhaften Variante der Erfindung eine Bewegung in Richtung des Anschlags (3) aus. Diese Bewegung wird durch Motorkraft gestützt. Auch hier erfolgt die Steuerung des Vorgangs durch die in die Prüfungsvorrichtung integrierte Recheneinheit. Gemäß dieser Erfindungsvariante ist der Anschlag (3) an einem Drehgelenk (8) mit Feder oder einem motorisierten Drehgelenk (8) befestigt. Im Falle eines Drehgelenks (8) mit Feder gibt der Anschlag (3) der Bewegung des Rechens (6a) nach. Nachdem der Rechen (6a) die Tablette (7) beziehungsweise deren Überreste von der Drehscheibe (1) geschoben hat, kehrt er in seine Ausgangsposition zurück. Durch die am Drehgelenk (8) anliegende Federkraft kehrt dabei auch der Anschlag (3) in seine Ausgangsposition zurück. Im Falle eines motorisierten Drehgelenks (8) wird der Anschlag durch Motorkraft bewegt, sodass er nicht mit dem Rechen (6a) in Berührung kommt, wenn dieser die Überreste der Tablette (7) von der Drehscheibe (1) schiebt. Wenn der Rechen (6a) in seine Ausgangsposition verfährt, wird auch die Ausgangsposition des Anschlags (3) durch Motorkraft wiederhergestellt. Erfindungsgemäß kann die Tablette (7) beziehungsweise können ihre Überreste auch auf andere Weise von der Drehscheibe (1) entfernt werden, beispielsweise mittels Druckluft,

Es ist vorgesehen, dass die Rotation der Drehscheibe (1) während der Prüfung fortgeführt wird oder die Rotation der Drehscheibe (1) ausgesetzt wird, sobald eine erfolgreiche Ausrichtung der Tablette (7) erfolgt ist.

Weiterhin kann der Rechen als Transportrechen (6) ausgestaltet sein, der geeignet ist, die Tablette (7) über eine Transportschiene (5) auf die Drehscheibe (1) zu schieben. Der Transport der Tablette (7) auf die Drehscheibe kann allerdings auch auf andere Weise erfolgen, beispielsweise kann die Tablette (7) der Drehscheibe (1) mittels einer Transportrinne zugeführt werden. Vorzugsweise ist der Transportrechen (6) darüber hinaus geeignet, die Funktion des vorangehend beschriebenen Rechens zu übernehmen, der zur Reinigung der Drehscheibe (1) eingesetzt wird.

In einer bevorzugten Ausführung des erfindungsgemäßen Verfahrens wird die Positionierung der Tablette (7) an mindestens einer Positionierungsfläche (2a, 3a) durchgeführt, welche eben oder konkav ist.

Weiterhin ist es bevorzugt, dass die auf die Drehscheibe (1) aufgebrachte Tablette (7) durch einen Wiegemechanismus gewogen wird, der Teil der Drehscheibe (1) ist.

Die erfindungsgemäße Vorrichtung und das erfindungsgemäße Verfahren werden nachfolgend anhand der Figuren 1 bis 4 näher erläutert.
Fig. 1 zeigt eine Ausführungsform der erfindungsgemäßen Vorrichtung. Eine besonders vorteilhafte Ausgestaltungsform der Erfindung wird in Fig. 2 bis Fig. 4 beschrieben. Es wird eine Kurzbeschreibung der Zeichnungen gegeben: Fig. 1 zeigt eine Draufsicht einer möglichen Ausführungsform in einem ersten Verfahrensschritt, umfassend eine Drehscheibe (1), eine erste Positionierungsfläche (2a), eine weitere Positionierungsfläche (3a), eine Bruchbacke (4), eine Transportschiene (5), einen Rechen (6a) und eine Tablette (7), wobei sich die Tablette (7) auf der Transportschiene (5) befindet.
Fig. 2 zeigt eine Draufsicht einer bevorzugten Ausführungsform in einem ersten Verfahrensschritt, umfassend eine Drehscheibe (1), ein Widerlager (2), einen Anschlag (3), eine Bruchbacke (4), eine Transportschiene (5), einen Transportrechen (6) und eine Tablette (7), wobei sich die Tablette (7) auf der Transportschiene (5) befindet.
Fig. 3 zeigt eine Draufsicht selbiger Ausführungsform wie in Fig. 2 in einem nachfolgenden Verfahrensschritt, wobei die Tablette (7) am Widerlager (2) ausgerichtet ist.
Fig. 4 zeigt eine Draufsicht selbiger Ausführungsform wie in Fig. 2 und Fig. 3 in einem weiteren Verfahrensschritt, wobei die Tablette (7) am Anschlag (3) ausgerichtet ist.

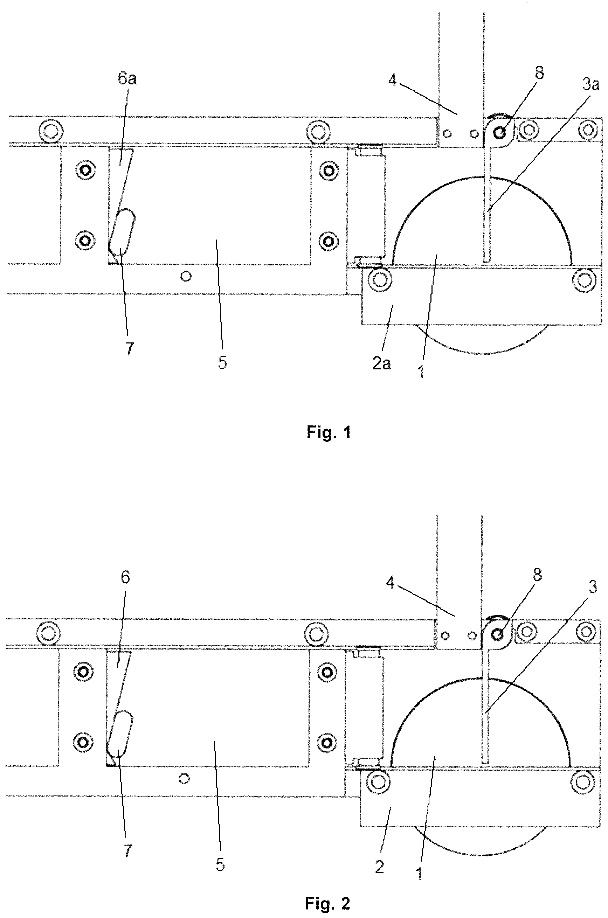

Die erfindungsgemäße Vorrichtung umfasst bevorzugt eine Drehscheibe (1), eine erste Positionierungsfläche (2a), eine weitere Positionierungsfläche (3a), eine Bruchbacke (4), eine Transportschiene (5), einen Rechen (6a) und eine Tablette (7), wobei sich die Tablette (7) in dieser Darstellung auf der Transportschiene (5) befindet (Fig. 1). Handelt es sich bei dem Rechen (6a) um einen Transportrechen (6), kann dieser die Tablette (7) auf die Drehscheibe (1) befördern.

Eine Tablette (7), vorzugsweise eine Oblong-Tablette, wird auf die Drehscheibe (1) verbracht. Die Tablette (7) kommt durch die Drehung der Drehscheibe (1) in einer ersten Richtung in Kontakt mit einer ersten Positionierungsfläche (2a), die vorzugsweise ein Widerlager (2) ist, und wird an dieser ausgerichtet. Die Ausrichtung der Tablette (7) erfolgt dadurch, dass diese durch die Drehung der Drehscheibe in Kontakt mit einer Positionierungsebene kommt und an dieser Positionierungsebene aufgrund der Drehung der Drehscheibe entlang ihrer Längsachse ausgerichtet wird. Dabei kann die Drehung der Drehscheibe (1) bereits erfolgen, bevor die Tablette auf die Drehscheibe gebracht wird, oder erst beginnen, nachdem sich die Tablette auf der Drehscheibe befindet. Anschließend findet mindestens eine Prüfung der Tablette (7) statt. Um eine weitere Prüfung durchzuführen, wird die Tablette (7) zur Prüfung in einer weiteren Position dadurch positioniert, dass die Drehscheibe in einer Richtung entgegengesetzt der Drehrichtung der vorherigen Drehung rotiert, sodass die Tablette (7) entlang einer Positionierungsfläche (3a), die vorzugsweise ein Anschlag (3) ist, ausgerichtet wird.

In einer bevorzugten Ausgestaltung der Vorrichtung ist ein Rechen (6a) vorgesehen, der das Tablettenmaterial nach erfolgter Prüfung von der Drehscheibe (1) schiebt. Handelt es sich bei dem Rechen (6a) um einen Transportrechen (6), kann dieser die Tablette (7) vor der Überprüfung auf die Drehscheibe (1) schieben. Der Transportrechen (6) kann somit sowohl die Tablette (7) auf die Drehscheibe (1) transportieren, als auch nach erfolgter Prüfung die Tablette (7) oder Tablettenreste von der Drehscheibe (1) schieben.

Nachfolgend wird anhand von Fig. 2-4 ein bevorzugtes Verfahren zur Prüfung von Tabletten gemäß der vorliegenden Erfindung beschrieben. Im Ausgangszustand befindet sich die Tablette (7) auf einer Transportschiene (5). Die Vereinzelung der Tabletten hat in einem vorigen Schritt stattgefunden, sodass sich stets nur eine Tablette (7) auf der Transportschiene (5) befindet. Der Transportrechen (6) vollzieht nun motorgestützt eine Bewegung in Richtung der Drehscheibe (1) und schiebt dadurch die Tablette (7) auf die Drehscheibe (1). Der Transportrechen (6) bewegt sich von der Drehscheibe (1) in Richtung seiner Ausgangsposition, jedoch lediglich so weit, dass er nicht mehr über der Drehscheibe (1) positioniert ist. Sobald die Tablette (7) auf der Drehscheibe (1) angelangt ist, beginnt die Drehscheibe (1) eine motorgestützte Rotation entgegengesetzt dem Uhrzeigersinn. Hierdurch wird die Tablette (7) entlang dem Widerlager (2) positioniert.

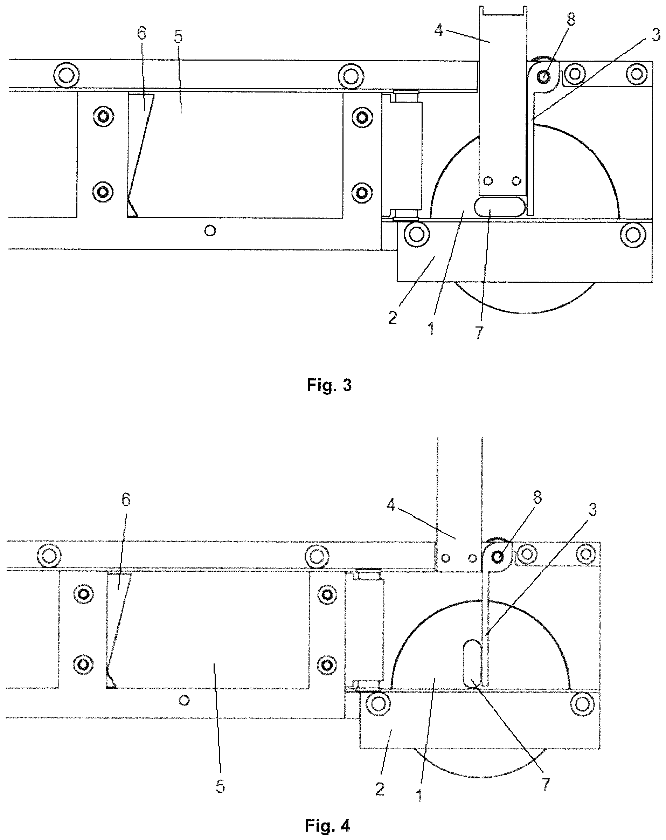

Während die Drehscheibe (1) fortlaufend entgegengesetzt dem Uhrzeigersinn rotiert, wird die Bruchbacke (4) so lange in Richtung des Widerlagers (2) bewegt, bis eine definierte Gegenkraft auftritt. Die zurückgelegte Strecke wird durch eine Recheneinheit, die in das Prüfgerät integriert ist und auch zur Steuerung der Bewegungsabläufe der Vorrichtung dient, aufgezeichnet. Dieser Verfahrensschritt wird in Fig. 3 dargestellt. Aufgrund der Ausrichtung der Tablette (7) wird hierbei deren Breite bestimmt.

Im Anschluss fährt die Bruchbacke (4) zurück in ihre Ausgangsposition und die Drehscheibe (1) beginnt eine Rotation in Richtung des Uhrzeigersinns. Hierdurch wird die Tablette (7) am Anschlag (3) ausgerichtet. Diesen Verfahrensschritt zeigt Fig. 4. Die Drehscheibe (1) setzt ihre Rotation fort, während sich die Bruchbacke in Richtung des Widerlagers (2) bewegt. Die Bewegung der Bruchbacke (4) wird ausgesetzt, sobald eine definierte Gegenkraft auf die Bruchbacke (4) auftritt. Die zurückgelegte Strecke wird durch die Recheneinheit aufgezeichnet. Aufgrund der Ausrichtung der Tablette (7) lässt sich aus der zurückgelegten Strecke die Länge der Tablette (7) bestimmen. Die Bruchbacke (4) verbleibt in ihrer aktuellen Position und legt nun eine Kraft in Richtung des Widerlagers (2) an, welche linear gesteigert wird. Sobald die Tablette (7) zu Bruch geht, wird die zu diesem Zeitpunkt angelegte Kraft durch die Recheneinheit aufgezeichnet. Die Drehscheibe (1) beendet ihre Rotation und die Bruchbacke (4) wird in ihre Ausgangsposition zurückbewegt.

Anschließend fährt der Transportrechen (6) in Richtung des Anschlags und schiebt dadurch die Überreste der Tablette (7) von der Drehscheibe (1). Hierbei trifft der Transportrechen (6) auf den Anschlag (3). Da er an einem Drehgelenk (8) befestigt ist, gibt der Anschlag (3) dem Transportrechen (6) nach. Der Transportrechen (6) kehrt nun in seine Ausgangsposition zurück. Hierbei nimmt auch der Anschlag (3) seine Ausgangsposition wieder ein, was durch eine im Drehgelenk verbaute Feder herbeigeführt wird. Eine weitere Tablette (7) kann nun auf die Transportschiene (5) platziert werden, sodass der Vorgang von neuem beginnen kann.

### Bezugszeichenliste

- 1.: Drehscheibe
- 2a.: Positionierungsfläche
- 2.: Widerlager
- 3a.: Positionierungsfläche
- 3.: Anschlag
- 4.: Bruchbacke
- 5.: Transportschiene
- 6a.: Rechen
- 6.: Transportrechen
- 7.: Tablette
- 8.: Drehgelenk

## Patentansprüche

1. Vorrichtung zur Prüfung von Tabletten, die über eine Drehscheibe (1) verfügt, die dazu eingerichtet ist, eine Tablette (7) zur Prüfung zu positionieren, **dadurch gekennzeichnet, dass** eine erste Positionierungsfläche (2a) oberhalb der Drehscheibe (1) als Widerlager (2) ausgeführt ist, entlang dem die Tablette (7) durch Rotation der Drehscheibe (1) in einer ersten Rotationsrichtung positionierbar ist, und eine zweite, an die erste Positionierungsfläche (2a) angrenzende Positionierungsfläche (3a) als Anschlag (3) ausgeführt ist, entlang dem die Tablette (7) durch Rotation der Drehscheibe (1) positionierbar und ausrichtbar ist, wenn die Drehrichtung der ersten Drehrichtung entgegengesetzt ist, und wobei die Vorrichtung über eine Bruchbacke (4) verfügt, die in Richtung des Widerlagers (2) bewegbar ist, wobei die Bruchbacke (4) dazu geeignet ist, den Durchmesser der Tablette zu messen, die entlang der mindestens einen Positionierungsfläche (2a, 3a) ausgerichtet ist, wobei der Durchmesser in Richtung der Bewegungsrichtung der Bruchbacke (4) gemessen wird.

2. Vorrichtung nach Anspruch 1, **dadurch gekennzeichnet, dass** Widerlager (2) und Anschlag (3) zueinander in einem Winkel von 90° ausgerichtet sind.

3. Vorrichtung nach Anspruch 1, **dadurch gekennzeichnet, dass** die Bruchbacke (4) dazu geeignet ist, eine Überprüfung der Bruchhärte einer Tablette (7) durchzuführen, die entlang der mindestens einen Positionierungsfläche (2a, 3a) ausgerichtet ist.

4. Vorrichtung nach einem der vorhergehenden Ansprüche, **dadurch gekennzeichnet, dass** ein Rechen vorgesehen ist, der das Tablettenmaterial nach erfolgter Prüfung von der Drehscheibe (1) schiebt.

5. Vorrichtung nach den Ansprüchen 1 und 4, **dadurch gekennzeichnet, dass** der Anschlag (3) an einem Drehgelenk (8) mit Feder befestigt ist, der dazu vorgesehen ist, einer Bewegung durch den Rechen nachzugeben und eigenständig in seine ursprüngliche Position zurückzukehren.

6. Vorrichtung nach Anspruch 4 oder Anspruch 5, **dadurch gekennzeichnet, dass** es sich bei dem Rechen um einen Transportrechen (6) handelt, mit dem die Tablette (7) vor der Überprüfung auf die Drehscheibe (1) schiebbar ist.

7. Vorrichtung nach einem der vorhergehenden Ansprüche, **dadurch gekennzeichnet, dass** die mindestens eine Positionierungsfläche (2a, 3a) als ebene oder konkave Fläche ausgestaltet ist und/oder eine Rechenanlage in die Prüfungsvorrichtung integriert ist, welche dazu vorgesehen ist, Abläufe zu steuern und gemessene Werte aufzuzeichnen.

8. Verfahren zur Prüfung von Tabletten, bei dem eine Tablette (7) auf eine Drehscheibe (1) aufgebracht wird, die Tablette (7) ausgerichtet wird, und anschließend mindestens eine Prüfung der Tablette (7) stattfindet, **dadurch gekennzeichnet, dass** zur Prüfung der Tablette (7) in einer ersten Position diese dadurch positioniert wird, dass die Drehscheibe (1) in einer ersten Richtung rotiert, sodass die Tablette (7) entlang einer Positionierungsfläche (2a), die ein Widerlager (2) ist, ausgerichtet wird und die Tablette (7) zur Prüfung in einer zweiten Position dadurch positioniert wird, dass die Drehscheibe in einer Richtung entgegengesetzt der ersten Drehrichtung rotiert, sodass die Tablette (7) entlang einer an die erste Positionierungsfläche (2a) angrenzenden Positionierungsfläche (3a), die ein Anschlag (3) ist, ausgerichtet wird, und die Prüfung der Tablette (7) durch eine Bruchbacke (4) durchgeführt wird, die eine Bewegung in Richtung des Widerlagers (2) vollzieht.

9. Verfahren nach Anspruch 8, **dadurch gekennzeichnet, dass** die Bruchbacke (4) die Prüfung des Durchmessers der Tablette (7) durchführt, die entlang der mindestens einen Positionierungsfläche ausgerichtet ist, indem sie eine Bewegung in Richtung des Widerlagers (2) so lange vollzieht, bis auf die Bruchbacke (4) eine Gegenkraft gemäß einem vordefinierten Wert auftritt.

10. Verfahren nach Anspruch 8 oder Anspruch 9, **dadurch gekennzeichnet, dass** die Bruchbacke (4) eine Prüfung der Bruchhärte einer Tablette (7) durchführt, die entlang der mindestens einen Positionierungsfläche (2a, 3a) ausgerichtet ist, indem sie eine Bewegung in Richtung des Widerlagers (2) so lange vollzieht, bis auf die Bruchbacke (4) eine Gegenkraft gemäß einem vordefinierten Wert auftritt, woraufhin die durch die Bruchbacke (4) angelegte Kraft in Bewegungsrichtung so lange gesteigert wird, bis ein Bruch der Tablette (7) auftritt.

11. Verfahren nach einem der Ansprüche 8 bis 10, **dadurch gekennzeichnet, dass** die Rotation der Drehscheibe während der Prüfung fortgeführt wird oder dass die Rotation der Drehscheibe ausgesetzt wird, sobald eine erfolgreiche Ausrichtung der Tablette erfolgt ist.

12. Verfahren nach einem der Ansprüche 8 bis 11, **dadurch gekennzeichnet, dass** ein Rechen das Tablettenmaterial nach erfolgter Prüfung von der Drehscheibe (1) schiebt.

13. Verfahren nach den Ansprüchen 8 und 12,**dadurch gekennzeichnet, dass** der Rechen in Richtung des Anschlags (3) geführt wird, der an einem Drehgelenk (8) mit Feder befestigt ist, und dass der Anschlag (3) durch Krafteinwirkung des Rechens beiseite gedrückt wird und durch die Federwirkung eigenständig in seine Ausgangsposition zurückkehrt, wenn der Rechen in seine Ausgangsposition verbracht wird.

14. Verfahren nach Anspruch 12 oder Anspruch 13, **dadurch gekennzeichnet, dass** der Rechen ein Transportrechen (6) ist, welcher die Tablette (7) vor der Prüfung auf die Drehscheibe (1) schiebt.

15. Verfahren nach einem der Ansprüche 8 bis 14, **dadurch gekennzeichnet, dass** die Positionierung an mindestens einer Positionierungsfläche durchgeführt wird, welche eben oder konkav ist und/oder dass eine integrierte Rechenanlage Abläufe steuert und gemessene Werte aufzeichnet.

## Claims

1. A device for testing tablets with a rotary disc (1) that is designed to position a tablet (7) for testing, **characterized in that** a first positioning surface (2a) is positioned above the rotary disc (1) as an abutment (2) along which the tablet (7) can be positioned by rotating the rotary disc (1) in an initial rotation direction and a second positioning surface (3a) that is located adjacent to the first positioning surface (2a) as an end stop (3) along which the tablet (7) can be positioned and aligned by rotating the rotary disc (1) when the direction of rotation is opposite to the first direction of rotation, and in which the device has a crusher jaw (4) that can be moved in the direction of the abutment (2), wherein the crusher jaw (4) is suitable for measuring the diameter of the tablet that is aligned along at least one of the positioning surfaces (2a, 3a), wherein the diameter is measured in the direction of the crusher jaw's (4) movement direction.

2. A device as set forth in Claim 1, **characterized in that** the abutment (2) and end stop (3) are oriented to one another at a 90° angle.

3. A device as set forth in Claim 1, **characterized in that** the crusher jaw (4) is suitable for testing the breaking resistance of a tablet (7) that is aligned along at least one of the positioning surfaces (2a, 3a).

4. A device as set forth in any one of the prior claims, **characterized in that** a rake is provided that pushes the tablet material from the rotary disc after testing (1).

5. A device as set forth in Claims 1 and 4, **characterized in that** the abutment (3) is mounted to a swivel (8) with a spring, which is intended to yield to a movement by the rake and autonomously return to its original position.

6. A device as set forth in Claim 4 or Claim 5, **characterized in that** the rake is a transport rake (6) with which the tablet (7) can be pushed onto the rotary disc (1) prior to testing.

7. A device as set forth in any one of the prior Claims, **characterized in that** at least one positioning surface (2a, 3a) is shaped as a flat or concave surface and/or a computer system is integrated into the testing device, which is provided to control processes and record measured values.

8. A process for testing tablets in which a tablet (7) is placed on a rotary disc (1), the tablet is aligned, and then the tablet (7) is tested at least once, **characterized in that**, for the purpose of testing the tablet (7) in an initial position, the tablet (7) is positioned by rotating the rotary disc (1) in an initial direction so that the tablet (7) is aligned along a positioning surface (2a) that is an abutment (2) and the tablet (7) is positioned for testing in a second position by rotating the rotary disc in the opposite direction of the initial rotation so that the tablet (7) is aligned along a positioning surface (3a), which is an end stop (3), that is adjacent to the first positioning surface (2a) and the tablet is tested by means of a crusher jaw (4) that moves in the direction of the abutment (2).

9. A process as set forth in Claim 8, **characterized in that** the crusher jaw (4) checks the diameter of the tablet (7), which is aligned along at least one of the positioning surfaces, by means of the crusher jaw (4) moving in the direction of the abutment (2) until a counterforce opposes the crusher jaw (4) in accordance with a predefined value.

10. A process as set forth in Claim 8 or Claim 9, **characterized in that** the crusher jaw (4) tests the breaking resistance of the tablet (7), which is aligned along at least one of the positioning surfaces (2a, 3a), by means of the crusher jaw (4) moving in the direction of the abutment (2) until a counterforce opposes the crusher jaw (4) in accordance with a predefined value, whereupon the force applied by the crusher jaw (4) increases in the direction of the movement until the tablet (7) fractures.

11. A process as set forth in any of Claims 8 through 10, **characterized in that** the rotation of the rotary disc is continued during the test or that the rotation of the rotary disc is suspended as soon as the tablet is successfully aligned.

12. A process as set forth in any of Claims 8 through 11, **characterized in that** a rake pushes the tablet material from the rotary disc after testing (1).

13. A process as set forth in Claims 8 and 12, **characterized in that** the rake is guided in the direction of the end stop (3), which is mounted to a swivel (8) with a spring, and that the end stop (3) is pushed aside by an application of force by the rake and autonomously returns to its starting position due to the spring effect, when the rake is placed in its starting position.

14. A process as set forth in Claim 12 or Claim 13, **characterized in that** the rake is a transport rake (6) that pushes the tablet (7) onto the rotary disc (1) prior to testing.

15. A process as set forth in any of the Claims 8 through 14, **characterized in that** the positioning is implemented on at least one positioning surface that is flat or concave and/or that an integrated computer system controls processes and records measured values.

## Revendications

1. Dispositif pour le contrôle de comprimés disposant d'une plaque tournante (1) qui est agencée pour positionner un comprimé (7) aux fins d'un contrôle, **caractérisé en ce qu'**une première surface de positionnement (2a) au-dessus de la plaque tournante (1) est exécutée comme culée (2), le long de laquelle le comprimé (7) peut être positionné dans un premier sens de rotation par la rotation de la plaque tournante (1), et une deuxième surface de positionnement (3a) adjacente à la première surface de positionnement (2a) est exécutée comme butée (3), le long de laquelle le comprimé (7) peut être positionné et orienté par la rotation de la plaque tournante (1), si le sens de rotation est inverse au premier sens de rotation, et où le dispositif est équipé d'une mâchoire de rupture (4) qui peut être déplacée dans le sens de la culée (2), où la mâchoire de rupture (4) est apte à mesurer le diamètre du comprimé qui est orienté le long d'au moins une des surfaces de positionnement (2a, 3a), où le diamètre est mesuré dans le sens du sens de déplacement de la mâchoire de rupture (4).

2. Dispositif selon la revendication 1, **caractérisé en ce que** la culée (2) et la butée (3) sont agencées dans un angle de 90° l'une par rapport à l'autre.

3. Dispositif selon la revendication 1, **caractérisé en ce que** la mâchoire de rupture (4) est apte au contrôle de la dureté de rupture d'un comprimé (7) qui est orienté le long d'au moins une des surfaces de positionnement (2a, 3a).

4. Dispositif selon une des revendications précédentes, **caractérisé en ce qu'**un râteau est prévu qui évacue le comprimé de la plaque tournante (1) après le contrôle réussi.

5. Dispositif selon les revendications 1 et 4, **caractérisé en ce que** la butée (3) est montée sur un pivot (8) à ressort qui doit céder un mouvement exécuté par le râteau et retourner à sa position initiale de manière autonome.

6. Dispositif selon la revendication 4 ou la revendication 5, **caractérisé en ce que** le râteau est un râteau de transport (6) apte à déplacer le comprimé (7) sur la plaque tournante (1) avant le contrôle.

7. Dispositif selon une des revendications précédentes, **caractérisé en ce qu' au** moins une des surfaces de positionnement (2a, 3a) est exécutée comme surface plane ou concave et/ou une installation de commande est intégrée dans le dispositif de contrôle qui doit commander les processus et enregistrer les valeurs mesurées.

8. Procédure pour le contrôle de comprimés où un comprimé (7) est placée sur une plaque tournante (1), le comprimé (7) est orienté et où par la suite au moins un contrôle du comprimé (7) a lieu, **caractérisée en ce que** le comprimé (7) est positionné pour le contrôle dans une première position par la rotation de la plaque tournante (1) dans un premier sens pour que le comprimé (7) soit orienté le long d'une surface de positionnement (2a), qui est une culée (2), et où le comprimé (7) est positionné pour le contrôle dans une deuxième position par la rotation de la plaque tournante dans un sens inverse du premier sens de rotation, pour que le comprimé (7) soit orienté le long d'une surface de positionnement (3a), qui est une butée (3), adjacente à la première surface de positionnement (2a) et où le contrôle du comprimé (7) est effectué par une mâchoire de rupture (4), qui effectue un mouvement dans le sens de la culée (2).

9. Procédure selon la revendication 8, **caractérisée en ce que** la mâchoire de rupture (4) effectue le contrôle du diamètre du comprimé (7), qui est orienté le long d'au moins une des surfaces de positionnement en exécutant un mouvement dans le sens de la culée (2) jusqu'à ce que la mâchoire de rupture (4) rencontre une force antagoniste répondant à une valeur prédéfinie.

10. Procédure selon la revendication 8 ou la revendication 9, **caractérisée en ce que** la mâchoire de rupture (4) effectue le contrôle de la dureté à la rupture du comprimé (7), qui est orienté le long d'au moins une des surfaces de positionnement (2a, 3a) en exécutant un mouvement dans le sens de la culée (2) jusqu'à ce que la mâchoire de rupture (4) rencontre une force antagoniste répondant à une valeur prédéfinie et la force appliquée par la mâchoire de rupture (4) dans le sens du mouvement est par la suite augmentée jusqu'à ce que la rupture du comprimé (7) survienne.

11. Procédure selon une des revendications 8 à 10 **caractérisée en ce que** la rotation de la plaque tournante est maintenue pendant le contrôle ou que la rotation de la plaque tournante est suspendue dès que l'orientation de comprimé a réussi.

12. Procédure selon une des revendications 8 à 11, **caractérisée en ce qu'**un râteau est prévu qui évacue le comprimé de la plaque tournante (1) après le contrôle réussi.

13. Procédure selon les revendications 8 et 12, **caractérisée en ce que** le râteau est guidé dans le sens de la butée (3), qui est montée sur un pivot (8) à ressort et que la butée (3) est repoussée par la force appliquée par le râteau et retourne à la position initiale de manière autonome par l'effet de ressort lorsque le râteau est amené à sa position initiale.

14. Procédure selon la revendication 12 ou la revendication 13, **caractérisée en ce que** le râteau est un râteau de transport (6) qui déplace le comprimé (7) sur la plaque tournante (1) avant le contrôle.

15. Procédure selon une des revendications 8 à 14, **caractérisée en ce que** le positionnement est exécuté à au moins une des surfaces de positionnement qui est plane ou concave et/ou qu'une installation de commande intégrée contrôle les procédures et enregistre les valeurs mesurées.
